# EUROPEAN PATENT SPECIFICATION

(11) **EP 2 456 871 B1**
(45) Date of publication and mention of the grant of the patent: **07.09.2016**
(21) Application number: 10802943.0
(22) Date of filing: 23.07.2010
(51) Int. Cl.: C12N 15/63, C12N 15/12, C12P 21/04, C07K 14/505, C12P 21/00, C12N 5/00

(54) **PRODUCTION OF ERYTHROPOIESIS STIMULATING PROTEIN USING METAL IONS**
HERSTELLUNG ERYTHROPOESE-STIMULIERENDER PROTEINE MITHILFE VON METALLIONEN
PRODUCTION D UNE PROTÉINE STIMULANT L ÉRYTHROPOÏÈSE, À L'AIDE D'IONS MÉTALLIQUES

(30) Priority: 24.07.2009 IN CH17412009
(43) Date of publication of application: 30.05.2012
(60) Divisional application: 15191348.0
(73) Proprietor: Dr. Reddy's Laboratories, Ltd., Hyderabad 500 016 AP (IN); Dr. Reddy's Laboratories Inc., Bridgewater, NJ 08807 (US)
(72) Inventor: PANKHANIA, Ashvin, 380 058 Gujarat (IN); REDDY, Degalahal Ganesh, 500 072 Andhra Pradesh (IN); KAMARAJU, Mamata, 500 079 Andhra Pradesh (IN)
(74) Representative: Bates, Philip Ian
(86) International application number: PCT/US2010/043037
(87) International publication number: WO 2011/011674

(56) References cited:
- WO-A1-2008/008360
- US-A1- 2007 161 084
- US-A1- 2007 161 084
- US-B1- 6 528 286
- CROWELL CHRISTOPHER K ET AL: "Amino acid and manganese supplementation modulates the glycosylation state of erythropoietin in a CHO culture system", BIOTECHNOLOGY AND BIOENGINEERING, WILEY & SONS, HOBOKEN, NJ, US, vol. 96, no. 3, 1 February 2007 (2007-02-01), pages 538-549, XP002454615, ISSN: 0006-3592, DOI: 10.1002/BIT.21141
- GAWLITZEK MARTIN ET AL: "Identification of cell culture conditions to control N-glycosylation site-occupancy of recombinant glycoproteins expressed in CHO cells.", BIOTECHNOLOGY AND BIOENGINEERING 15 AUG 2009, vol. 103, no. 6, 9 April 2009 (2009-04-09) , pages 1164-1175, XP002688571, ISSN: 1097-0290
- BORK K ET AL: "Increasing the sialylation of therapeutic glycoproteins: the potential of the sialic acid biosynthetic pathway", JOURNAL OF PHARMACEUTICAL SCIENCES, AMERICAN PHARMACEUTICAL ASSOCIATION, WASHINGTON, US, vol. 98, no. 10, 6 February 2009 (2009-02-06), pages 3499-3508, XP002572996, ISSN: 0022-3549, DOI: 10.1002/JPS.21684 [retrieved on 2009-02-06]
- YANG AND M BUTLER M: "Effect of Ammonia on the Glycosylation of Human Recombinant Erythropoietin in Culture", BIOTECHNOLOGY PROGRESS, AMERICAN INSTITUTE OF CHEMICAL ENGINEERS, US, vol. 16, no. 5, 1 January 2000 (2000-01-01), pages 751-759, XP008150310, ISSN: 8756-7938, DOI: 10.1021/BP000090B [retrieved on 2000-09-19]
- SINCLAIR ANGUS M ET AL: "Glycoengineering: the effect of glycosylation on the properties of therapeutic proteins", JOURNAL OF PHARMACEUTICAL SCIENCES, AMERICAN PHARMACEUTICAL ASSOCIATION, WASHINGTON, US, vol. 94, no. 8, 1 August 2005 (2005-08-01) , pages 1626-1635, XP002559550, ISSN: 0022-3549
- RICH JAMIE R ET AL: "Emerging methods for the production of homogeneous human glycoproteins.", NATURE CHEMICAL BIOLOGY APR 2009, vol. 5, no. 4, April 2009 (2009-04), pages 206-215, XP002688572, ISSN: 1552-4469
- WITSELL D L ET AL: "DIVALENT CATION ACTIVATION OF GALACTOSYLTRANSFERASE IN NATIVE MAMMARY GOLGI VESICLES", JOURNAL OF BIOLOGICAL CHEMISTRY, AMERICAN SOCIETY FOR BIOCHEMISTRY AND MOLECULAR BIOLOGY, US, vol. 265, no. 26, 15 September 1990 (1990-09-15), pages 15731-15737, XP002454614, ISSN: 0021-9258
- CHRISTOPHER, K. ET AL.: 'Amino acid and manganese supplementation modulates the glycosylation state of erythropoietin in a CHO culture system.' BIOTECHNOL. BIOENG. vol. 96, 2007, pages 538 - 549, XP002454615
- YANG, M. ET AL.: 'Effect of ammonia on the glycosylation of human recombinant erythropoietin in culture.' BIOTECHNOL. PROG. vol. 16, 2000, pages 751 - 759, XP008150310

## Description

### INTRODUCTION

This invention relates to culture methods for the production of erythropoiesis stimulating proteins.

Erythropoiesis stimulating proteins, such as erythropoietin and analogs of erythropoietin are glycoprotein hormones that are the principle homeostatic regulators of red blood cell production. Natural erythropoietin, or EPO, is produced by the kidney; but its high demand for therapeutic purposes has led to large scale production by recombinant DNA methods. Purified recombinant human EPO is administered to human patients for the treatment of medical indications associated with inadequate red blood cell supply, such as anemia and chronic renal failure. Recombinant human erythropoietin (rHuEPO) and its analog darbepoetin alfa are used clinically to treat anemia and increase red blood cell production in various conditions, such as perisurgery, chronic renal failure, side effects of HIV or HCV treatment, and side effects of cancer chemotherapy.

The recombinant expression and production of glycoproteins, such as EPO and analogs of EPO, is complicated by the need for both high level expression, as well as appropriate post translation processing of these glycoproteins. An important post-translational processing that occurs subsequent to cellular protein expression is glycosylation, wherein glycans are enzymatically attached to the polypeptide backbone. Protein glycosylation is of two general types: O-linked glycosylation, wherein the glycan group is attached to the hydroxyl oxygen of serine or threonine side chains; and N-linked glycosylation, wherein the glycan group is attached to amide nitrogen of aspargine side chains. The nature and composition of the sugars that makeup the glycan group can significantly affect the physico-chemical properties, isoform composition and biological activities of glycoproteins. For instance N-acetylneuraminic acid (hereinafter referred to as sialic acid) is one of the most commonly present sugars in the glycan moiety of glycoproteins. Being negatively charged, the number of sialic acid groups present in a glycoprotein glycan affects the isoelectric point of the glycoprotein: more the number sialic acids, lower the isoelectric point. Darbepoetin alfa, a novel glycosylation analog of rHuEPO contains five N-linked carbohydrate chains and up to 22 sialic acids. In contrast, recombinant human EPO has 3 N-linked carbohydrate chains and a maximum of 14 sialic acids (Egrie JC., and Browne JK., Br. J. cancer 2001; 84, 3-10 and Elliot S, et.al., Blood 2000;96:8 2a). As a result of the additional glycosylation (and sialylation), darbepoetin has decreased receptor-binding activity, but exhibits a three-fold longer serum half-life and increased in vivo activity as compared to recombinant human EPO.

rHuEPO produced in Chinese hamster ovary (CHO) cells can exhibit a variable extent of glycosylation and sialylation. (Takeuchi et al., 1989 PNAS 86(20):7819-22, Zanette et al., 2003 Journal of Biotechnology 101 (3):275-287). Given that sialylation of erythropoietin is necessary for its *in vivo* bioactivity, consistency in glycosylation and higher levels of sialylation of rHuEPO and its analogs are desirable attributes when producing these recombinant proteins for therapeutic purposes. Thus there is a need for methods that improve glycosylation and sialylation of these glycoproteins in cell cultures.

The literature describes methods for increasing glycosylation of glycoproteins by culturing cells expressing the same in media comprising metal ions. U.S. Patent No. 6,528,286 discloses a process for the preparation of glycoprotein in which the sialic acid content in glycoprotein is increased by addition of copper ions to the cell culture. However, the patent does not describe the use of divalent manganese ions or trivalent iron ions.

U.S. Patent Application Publication No. 2007/0161084 describes a method for producing erythropoietic compositions with some increase in sialylation by culturing cell lines expressing an erythropoietic protein in medium comprising 0.01 to 50 µM concentration of manganese. However the application reports increase in the toxicity for cells at concentrations greater than 60 µM.

### Summary

The present invention provides a process for increasing the levels of low pl isoforms of darbepoetin alfa, comprising culturing Chinese hamster ovary cell lines expressing said darbepoetin alfa, in a medium comprising divalent manganese ions having concentrations in a range of 60 µM to 200 µM wherein the darbepoetin alfa so obtained demonstrates an increase in low pl isoforms in comparison to darbepoein alfa obtained from cells cultured in absence of said concentration of Manganese as determined by isoelectric focusing.

The culture medium is optionally serum-free and may comprise one or more supplementary amino acids such as aspargine, aspartic acid, cysteine, cystine, isoleucine, leucine, tryptophan or valine. The host cells may be grown in any suitable cell culture systems such as in roller bottles.

### BRIEF DESCRIPTION OF THE DRAWINGS

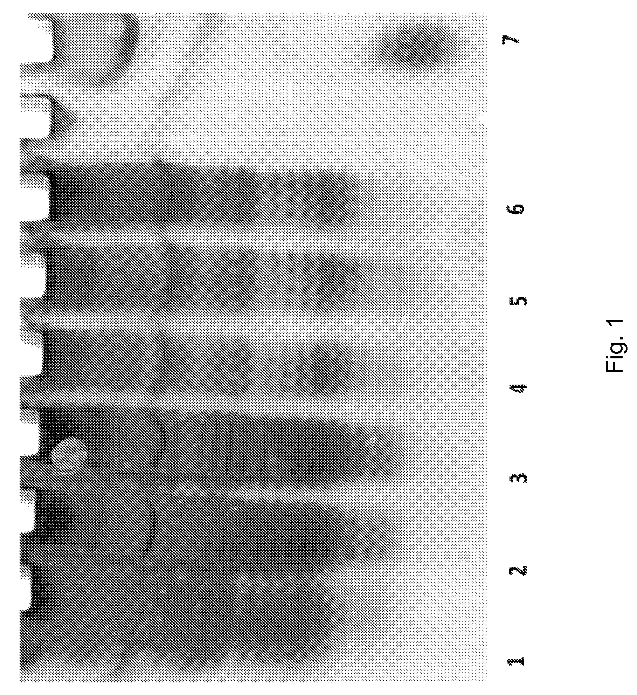

Fig. 1 shows an IEF gel, performed as described in Example 2, of harvest of cells cultured in medium devoid of either manganese ions or trivalent iron ions (Example 1A). Lanes 1-6 are different harvest batches. Lane 7 is a sample of Aranesp® used as control. The isoforms of Aranesp® have a pl range of 3-3.9 *(see* Franc oise Lasne et al., Analytical Biochemistry 311 (2002) 119-126).

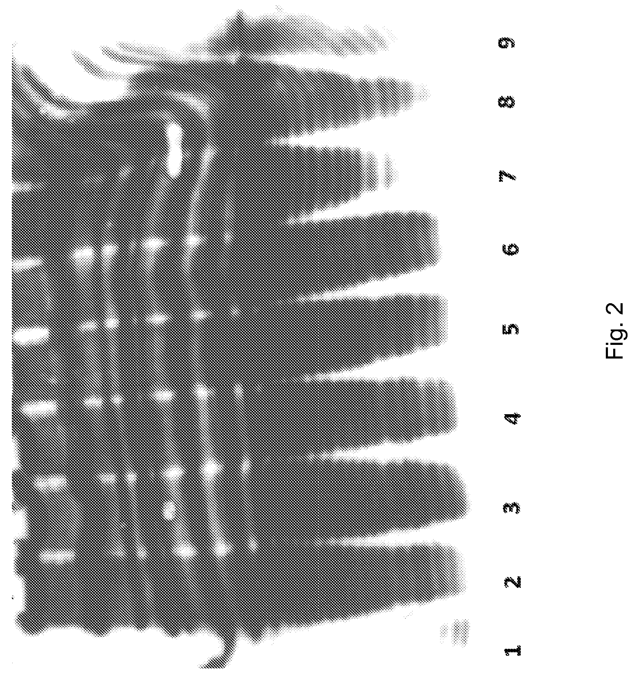

Fig. 2 shows an IEF Gel gel, performed as described in Example 2, of harvest of cells cultured in medium supplemented with 100 µM manganese chloride but devoid of trivalent iron ions. Lane 1 is Aranesp® and lanes 2-9 are the darbepoetin alfa samples showing low pl isoforms expressed in the presence of manganese chloride.

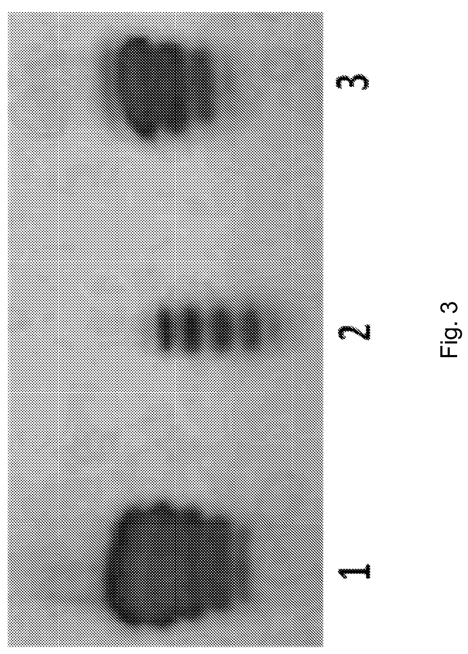

Fig. 3 shows an IEF gel performed as described in Example 2 showing the low pl isoforms of darbepoetin alfa. Lane 1 is harvests of cells grown in the presence of 80 µM manganese and lane 3 is with 38 µM ferric ammonium citrate, subjected to purification by methods disclosed in International Application No. PCT/US2009/048239 and incorporated herein by reference. Purified samples were then analysed by IEF. Aranesp® (Lane 2) was used as a control.

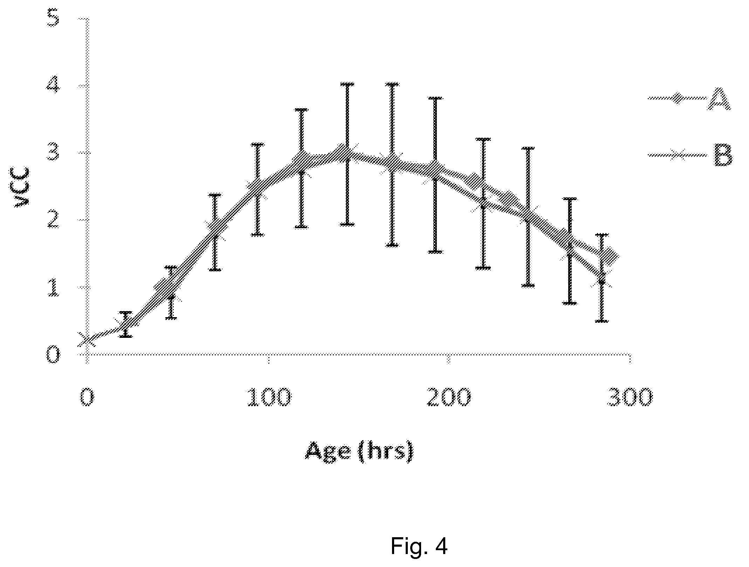

Fig. 4 shows a profile showing the viable cell count and percentage viability against age. Line A represents the VCC and percentage viability of cells cultured in the absence of manganese chloride. Line B represents the VCC and percentage viability of cells cultured in the presence of manganese chloride. Lines A and B show similar viable cell count patterns indicating that higher concentrations of manganese chloride do not affect the viable cell count, and hence is not cytotoxic.

### DETAILED DESCRIPTION

The term "isoform", as used herein, refers to proteins with identical amino acid sequence that differs with respect to charge and therefore isoelectric point, as a result of differences in glycosylation, acylation, deamidation, or sulfation.

The "isoelectric point" or "pI" is the pH at which a particular molecule or surface carries no net electrical charge. The "pI" of a polypeptide refers to the pH at which the polypeptide's positive charge balances its negative charge. The pI can be estimated by various methods known in the art, e.g., from the net charge of the amino acid and/or sialic acid residues on the polypeptide or by using isoelectric focusing, chromatofocusing, etc.

The "low pI isoforms" refer to isoforms with the pI of about 5 or less.

"Non toxic amount or levels" refers to the concentration of metal ions (such as manganese or trivalent ferric ions) in the cell culture media at which there is no reduction in the cell viability, cell growth, or protein production, as compared to media devoid of metal ions.

Erythropoiesis-stimulating proteins, such as human erythropoietin (EPO) and analogs of erythropoietin are glycoprotein hormones that are the principle homeostatic regulators of red blood cell production and are used in the treatment of anemia caused by chronic renal failure.

Darbepoetin alfa, an EPO analog with five N-linked carbohydrate chains and up to 22 sialic acids, exhibits a three-fold longer serum half-life and increased *in vivo* activity as compared to recombinant human EPO. Compositions of erythropoiesis stimulating proteins, such as darbepoetin alfa, comprise a mixture of various isoforms that are known to differ in their extent of glycosylation and sialylation. The low pl isoforms of rHuEPO and darbepoetin alfa, as result of higher sialic acid content, exhibit much higher specific activity as compared to isoforms of higher pl (having low sialic acid content) (*e.g*., N. Imai et al., "Physicochemical and Biological Characterization of Asialoerythropoeitin," European Journal of Biochemistry Vol. 194 (2), pages 457-462, 1990; and EP-A-0 428 267). Hence isoforms with higher sialic acid content and lower pl are of greater therapeutic value. For example, Aranesp® (Amgen Corporation), the approved and marketed form of darbepoietin alpha, comprises essentially low pl isoforms of the protein, in the pl range 3.0-3.9 (see F. Lasne et al., "Detection of isoelectric profiles of erythropoetin in urine:differentiation of natural and administered recombinant hormones," Analytical Biochemistry, Vol. 311 (2), pages 119-126, 2002).

Exemplary sequences, manufacture, purification and use of darbepoetin and other erythropoietin analogs are described in a number of patent publications, including, and International Application Publications WO 91/05867 of Strickland et al., WO 95/05465 of Elliott et al., WO 00/24893 of Egrie et al., and WO 01/81405 of Egrie et al., each of which is incorporated herein by reference in its entirety.

The cell culture medium comprises high levels of divalent manganese (Mn⁺²) ions, which are effective in increasing the levels of low pl isoforms or erythropoiesis stimulating protein without affecting the viability of the cell culture.

The addition of high concentrations of divalent manganese ions and/or trivalent iron ions to the culture medium results in an increase in the number and yield of low pI isoforms of erythropoiesis-stimulating molecules, such as human erythropoietin, or its biologically active variants, derivatives (including chemically modified derivatives), or analogs, such as darbepoetin.

U.S. Patent Application Publication No. 2007/0161084 discloses the use of Mn⁺² ions in a culture medium at concentrations of 0.01 µM to 50 µM to improve the yield of erythropolesis stimulating protein. However, toxicity and poor protein yield is reported at concentrations greater than 50 µM. In contrast, the present invention disdoses processes of increasing the levels of low pl isoforms of erythropolesis stimulating protein by growing cell cultures expressing erythropolesis stimulating proteins in a culture medium comprising Mn⁺² without any concomitant decrease in cell viability.

The present invention provides a process for increasing the levels of low pl isoforms of darbepoetin alfa, comprising culturing Chinese hamster ovary cell lines expressing said darbepoetin alfa, in a medium comprising divalent manganese ions having concentrations in a range of 60 µM to 200 µM wherein the darbepoetin alfa so obtained demonstrates an increase in low pl isoforms in comparison to darbepoein alfa obtained from cells cultured in absence of said concentration of Manganese as determined by isoelectric focusing.

In embodiments, the concentration of Mn⁺² ions in the culture medium ranges from about 60 µM to about 200 µM, or about 70 µM to about 160 µM, or is about 100 µM.

Examples of useful salts of divalent manganese ions include, but are not limited to, manganese sulphate and manganese chloride.

Salts of divalent manganese ions at high non-toxic concentration are effective in increasing the levels of low pl isoforms of erythropoiesis stimulating protein and but does not reduce the cell viability, cell growth or protein.

Without wishing to be bound by any particular theory, the use of the selenium, poloxamers, or both in a cell culture medium may prevent adverse effects of high concentrations of Mn⁺² ions on the cells, thereby maintaining cell viability.

Thus, in embodiments, the cell culture media comprise selenium, or salts of selenium such as sodium selenite.

In embodiments, the cell culture media comprise a poloxamer such as Pluronic® F68.

In embodiments, the cell culture media comprise sodium selenite and a poloxamer such as Pluronic® F 68.

The culture media can also comprise any other nutrient ingredient known in the art, such as carbohydrates, including glucose, essential and non-essential amino acids, lipids and lipid precursors, nucleic acid precursors, vitamins, inorganic salts, trace elements including rare metals, and cell growth factors. A culture medium may be a chemically defined medium or may include serum, plant hydrolysates, or other undefined substances. The culture medium may be entirely serum-free or free of any animal-component. Exemplary media used for culturing the cells include DMEM/F-12 (GIBCO®) media about 15.6 g/L. DMEM medium comprises the following inorganic salts: Calcium Chloride, Cupric sulfate, Potassium Chloride, Magnesium Sulfate or Chloride, Sodium Chloride, Sodium Dihydrogen Phosphate, Sodium Bicarbonate, Zinc sulfate; the following amino acids L-Alanine, L-Arginine, L-Asparagine, L-Aspartic acid, L-Cysteine, L-glutamic acid, L-Glutamine, Glycine, L-Histidine, L-Isoleucine, L-Leucine, L-Lysine, L-Methionine, L-Phenylalanine, L-Proline, L-Serine, L-Threonine, L-Tryptophan, L-Tyrosine, L-Valine; the following lipids and vitamins: Biotin, D-Calcium-Pantothenate, Choline Chloride, Folic Acid, myo-Inositol, Niacinamide, Nicotinamide, Pyridoxine, Riboflavin, Thiamine, Vitamin B12 (cobalamin), Thymidine, Linoleic Acid, Lipoic Acid; and other components including D-Glucose, Phenol Red, Hypoxanthine, Sodium Pyruvate, Putrescine (1,4-diamino butane), and HEPES (4-(2-hydroxyethyl)-1-piperazineethanesulfonic acid). The culture medium may also include supplementary amino acids, e.g. asparagine, aspartic acid, cysteine, cystine, isoleucine, leucine, tryptophan, and valine. The medium may also comprise lipids and/or lipid precursors such as ethanolamine 5.1 µg/L, Soy peptone 1.0 g/L, Wheat peptone 1 g/L, Fructose 1.0 g/L, sodium bicarbonate 1.2 g/L, Pluronic® F-68 1.0 g/L, HyQ® (HyClone Cell Boost™ 2, Catalogue# SH30890) 35 mL/L, sodium selenite 5 µg/L, and insulin 5 mg/L. In an embodiment, the addition of MnCl₂ is done after a period of rapid growth phase of about 9 days along with the HyQ® feed of about 35 mL/L.

The following examples will further illustrate certain specific aspects and embodiments of the invention in greater detail, and are not intended to limit the scope of the invention.

### EXAMPLE 1

### Preparation of the clone

Darbepoetin alfa producing cell lines were made by transduction of the CHO-S parental cell line with expression retrovector encoding gene of darbepoetin alfa. Following transduction, the pooled population of cells expressed up to 36 µg/ml of darbepoetin alfa after 10-14 days in T-flasks. The pooled population of cells were diluted to very low cell density (1-3 viable cells/200 µL of medium) and plated in 96 well microtiter plates to establish clonal cell lines that originated from single cells. Clones were screened for darbepoetin alfa production and the clones with high productivity were selected for expression.

### COMPARATIVE EXAMPLE 1A

### Expression of darbepoetin alfa in the absence of either manganese or trivalent ferric ions

The cells expressing darbepoietin alpha were expanded in spinners and seed reactor before being inoculated into the production reactor. PF CHO HyClone® medium (Catalog # SH30333.03) was used for culturing the cells in the spinner bottle. Table 1 shows the composition of PF CHO medium used for culturing cells. The pH of the medium was 7 and cells from the master cell bank were inoculated at an initial cell count of about 0.2 million cells/mL. The spinner bottles were incubated in a 5% CO₂ and at 37°C for 72 hours. After the spinner stages, cells were inoculated in a 6.5 L seed reactor containing 4 L of SFM-6 medium (composition in Table 2) at an initial cell density of 0.2 million cells/mL, the pH was maintained at 7, temperature at 37.0°C and dissolved oxygen was maintained at 40%. After 72 hours the seed reactor culture was aseptically harvested and the cells were transferred to an 11.5 L production reactor containing 10 L of SFM-6 medium at an initial cell density of 0.2 million cells/mL and fermentation continued under conditions maintained as in the seed reactor described above. The production culture was harvested after 12 days, and supernatant was collected and analyzed.

**Table 1: Composition of (Hyclone®) PF-CHO medium**

| | |
|---|---|
| PF-CHO main powder | 6.0 g, |
| PF-CHO base powder | 10.4 g |
| L-Glutamine | 0.58 g |
| Pluronic® F 68 | 1.0 g |
| Sodium bicarbonate | 2.0 g |
| pH | 7.0 |

| | |
|---|---|
| * PLURONIC F 68 is a copolymer of ethylene oxide and propylene oxide, having an average molecular weight of 8400, and is sold by BASF (Germany). | |

**Table 2: Composition of SFM-6 medium**

| | |
|---|---|
| DMEM/F-12 | 15.6 g |
| MEM amino acids solution (60X) | 10 mL |
| MEM vitamin solution (100X) | 10 mL |
| TE 30 (1000X) | 1 mL |
| Sodium selenite | 5 µg |
| Ethanolamine | 5.1 µg |
| Soy peptone | 1.0 g |
| Wheat peptone | 1.0 g |
| Fructose | 1.0 g |
| Sodium bicarbonate | 1.2 g |
| Pluronic F-68 | 1.0 g |
| HyQ® | 35 mL |
| Insulin | 5.0 mg |

### EXAMPLE 1B:

### Expression of darbepoetin alfa in the presence of manganese

The culture medium in the seed reactor stage and production stage were supplemented with manganese chloride to a final concentration of 80 to 100 µM. All other conditions were maintained as described in Example 1A. The cell viability so obtained was compared to the viability obtained in Example 1 A (see Fig. 3 and Fig. 4).

### COMPARATIVE EXAMPLE 1C

### Expression of darbepoetin alfa in the presence of trivalent iron ions.

The culture medium in the seed reactor stage and production stage were supplemented with ferric ammonium citrate to a final concentration of 38 µM. All other conditions were maintained as described in Example 1 A.

### EXAMPLE 2

### Isoform analysis by IEF

Harvests from comparative Examples 1A and 1C and Example 1B were analysed by IEF for the expression of darbepoetin alfa. The gel for IEF was prepared using water, urea, 30% acrylamide, and ampholyte (pH range 2-4 and 3-10). The above components were mixed gently and 10% w/v ammonium per sulphate and TEMED (Tetramethylethylenediamine) were added to mixture and the mixture was casted in gel sandwich apparatus (Bio-Rad Mini-PROTEAN® Cell) and fitted with a comb. The gel was allowed to polymerize for 45 minutes at room temperature. A small amount of this sample was mixed with equal volume of sample buffer (glycerol, ampholyte and Milli Q water) and loaded into the gel. The gel was then placed in Bio-Rad Mini-PROTEAN® Cell assembly and filled with cathode buffer (25 mM sodium hydroxide) and anode buffer (25 mM ortho phosphoric acid) in separate compartments. Samples were run at 200 V for 1.5 hours for pre-focusing of ampholytes at room temperature and then the voltage was increased to 400 V and run for the next 1.5 hours at room temperature. After the run, the gel was carefully removed and stained using either a Coomassie stain or Silver stain. The samples were analyzed (see Fig. 2 and Fig. 3).

## Claims

1. A process for increasing the levels of low pl isoforms of darbepoetin alfa, comprising culturing Chinese hamster ovary cell lines expressing said darbepoetin alfa, in a medium comprising divalent manganese ions having concentrations in a range of 60 µM to 200 µM wherein the darbepoetin alfa so obtained demonstrates an increase in low pl isoforms in comparison to darbepoein alfa obtained from cells cultured in absence of said concentration of Manganese as determined by isoelectric focusing.

2. A process according to claim 1, wherein a concentration of divalent manganese ions is 70 µM to 160 µM.

3. A process according to claim 1, wherein a concentration of divalent manganese ions is 100 µM.

4. A process according to claim 1, wherein the divalent manganese ions are derived from salts of manganese.

5. A process according to any of claims 1-4, wherein the divalent manganese ions are derived from MnCl₂.

6. A process according to any of claims 1-8, wherein a medium further comprises selenium or a salt of selenium.

7. A process according to claim 10, wherein a salt of selenium is sodium selenite.

8. A process according to any of claims 1-8, wherein a medium further comprises a poloxamer.

9. A process according to any of claims 1-8 wherein a medium further comprises selenium or a salt of selenium, and a poloxamer.

## Patentansprüche

1. Verfahren zum Erhöhen der Level von Isoformen von Darbepoetin alfa mit niedrigem pI, das Folgendes beinhaltet: Kultivieren von Chinesischer-Hamster-Ovarienzelllinien, die das genannte Darbepoetin alfa exprimieren, in einem Medium, das zweiwertige Manganionen mit Konzentrationen im Bereich von 60 µM bis 200 µM enthält, wobei das so erhaltene Darbepoetin alfa eine Zunahme von Isoformen mit niedrigem pI im Vergleich zu Darbepoetin alfa aufzeigt, das von Zellen erhalten wird, die ohne die genannte Konzentration von Mangan kultiviert werden, wie durch isoelektrische Fokussierung bestimmt.

2. Verfahren nach Anspruch 1, wobei eine Konzentration von zweiwertigen Manganionen 70 µM bis 160 µM beträgt.

3. Verfahren nach Anspruch 1, wobei eine Konzentration von zweiwertigen Manganionen 100 µM beträgt.

4. Verfahren nach Anspruch 1, wobei die zweiwertigen Manganionen von Salzen von Mangan stammen.

5. Verfahren nach einem der Ansprüche 1-4, wobei die zweiwertigen Manganionen von MnCl₂ stammen.

6. Verfahren nach einem der Ansprüche 1-8, wobei ein Medium ferner Selen oder ein Salz von Selen beinhaltet.

7. Verfahren nach Anspruch 10, wobei ein Salz von Selen Natriumselenit ist.

8. Verfahren nach einem der Ansprüche 1-8, wobei ein Medium ferner ein Poloxamer beinhaltet.

9. Verfahren nach einem der Ansprüche 1-8, wobei ein Medium ferner Selen oder ein Salz von Selen und ein Poloxamer beinhaltet.

## Revendications

1. Procédé destiné à augmenter les niveaux d'isoformes à faible pI de darbépoétine alfa, comprenant cultiver des lignées cellulaires ovariennes de hamster chinois exprimant ladite darbépoétine alfa, dans un milieu comprenant des ions divalents de manganèse ayant des concentrations dans la plage de 60 µM à 200 µM dans lequel la darbépoétine alfa ainsi obtenue démontre une augmentation en isoformes à faible pI par comparaison à de la darbépoétine alfa obtenue de cellules cultivées en l'absence de ladite concentration de manganèse telle que déterminée par focalisation isoélectrique.

2. Procédé selon la revendication 1, dans lequel une concentration d'ions divalents de manganèse est de 70 µM à 160 µM.

3. Procédé selon la revendication 1, dans lequel une concentration d'ions divalents de manganèse est de 100 µM.

4. Procédé selon la revendication 1, dans lequel les ions divalents de manganèse sont dérivés de sels de manganèse.

5. Procédé selon l'une quelconque des revendications 1-4, dans lequel les ions divalents de manganèse sont dérivés de MnCl₂.

6. Procédé selon l'une quelconque des revendications 1-8, dans lequel un milieu comprend en outre du sélénium ou un sel de sélénium.

7. Procédé selon la revendication 10, dans lequel un sel de sélénium est de la sélénite de sodium.

8. Procédé selon l'une quelconque des revendications 1-8, dans lequel un milieu comprend en outre un poloxamère.

9. Procédé selon l'une quelconque des revendications 1-8, dans lequel un milieu comprend en outre du sélénium ou un sel de sélénium et un poloxamère.
